# EUROPEAN PATENT APPLICATION

(11) **EP 1 037 089 A1**
(43) Date of publication of application: **20.09.2000**
(21) Application number: 98950535.9
(22) Date of filing: 22.09.1998
(51) Int. Cl.: G02C 1/00, G02C 7/16, A61F 9/00

(54) **PERSONAL ACCESSORY FOR WORKING WITH THE SCREEN OF AN ELECTRONIC DEVICE**

(30) Priority: 07.10.1997 RU 97116445; 04.09.1998 RU 98116467
(71) Applicant: Toganov, Vladimir Mihaylovich, Tverskaya obl., 172063 (RU)
(72) Inventor: Toganov, Vladimir Mihaylovich, Tverskaya obl., 172063 (RU)
(74) Representative: Einsel, Martin, Dipl.-Phys.
(86) International application number: RU9800300
(87) International publication number: WO9918470

(57) **Abstract**

The present invention relates to an accessory for working with electronic devices that include screens for representing images, texts, letters, etc. This accessory comprises a system for fixing it to the head of a user, wherein said system is connected to a device for releasing the tension of the eye muscles. This device is made in the shape of at least one member for blocking the field of vision of both eyes, wherein said member is made of light absorbing and/or scattering material. This device is used for blocking sequentially the user's left and right eyes in order to relax the weakening muscles of both eyes and thus increase the user's working capacity. This accessory is easy to manufacture and to use and has a reduced cost.

## Description

### FIELD OF THE INVENTION

The present invention relates to an accessory for working with displays, computer monitor screens, TV-sets and other electronic devices.

### BACKGROUND OF THE INVENTION

From the background of the invention an accessory is known for blocking the field of vision of eyes, comprising a system for fixing it to the head of a user, made in the shape of a spectacles rim, and a member for blocking one of the eyes (US 4582401A, Cl. G02C 7/16, 1986). Field of using this accessory is limited by squint correction.

Also a personal accessory is known for working with the screen of a computer display, comprising a system for fixing it to the head of a user, made in the shape of a spectacles rim, connected to a device for releasing the tension of the eye muscles, made in the shape of an optical protective filter, covering the surface of glasses lens (RU 2005314 Cl, Cl. G02B 5/20, 1993).

The last accessory is chosen as the nearest analogue of the invention. Despite the fact, that the known accessory as well as the proposed one is aimed at increasing the working capacity of the user, working with the screen of an electronic device owing to decreasing of the fatigue of his eyes, the known accessory does not provide relaxing the weakening muscles of both eyes.

### SUMMARY OF THE PRESENT INVENTION

The technical result of the invention consists in increasing the efficiency and the duration of the user's working with the screens of the abovementioned devices owing to relaxing the weakening muscles of both eyes and also in making it easy to manufacture and to use and also in reducing its cost significantly.

The summary of the invention consists in the fact, that as well as in the nearest analogue, the personal accessory for working with the screen of an electronic device comprises a system for fixing it to the head of a user, connected to a device for releasing the tension of the eyes, but in contradistinction to the nearest analogue, the device for releasing the tension of the eyes is made in the shape of at least one member for blocking the field of vision of the eyes, which provides the possibility of blocking sequentially the user's left and right eyes. The said member may be made in the shape of a flap or a shield, made of light absorbing and/or scattering material.

The accessory is characterized also with that the system for fixing it to the head of a user may be made in the shape of an elastic bandage, a hoop, a helmet or a spectacles rim. In the case of a rigid system for fixing to the head of a user, at least one sheild may be joint with it by means of a hinge.

When the user looks with both eyes, he receives a volumetric image, which permits to orient oneself and to act easier in the surrounding.

Texts and drawings on the paper, images on the display, monitor screen, TV-set are plane by their physical nature. Even if an illusion of a volume is created with the help of various methods (perspective, color effects etc.), it is not connected with the perception with two eyes, these effects remain during perception with one eye.

Therefore the process of reading, writing, working and leisure with the help of a display, monitor of TV-set by means of one eye is possible and sufficient.

The advantage of the invention consists in following: the weakening muscles of the eyes, which are usually tensed, while blocking the field of vision of one of the eyes relax, the tension created in the eyes is taken off, and the user becomes capable to work longer and more effectively without fatigue and ache in the eyes.

### BRIEF DESCRIPTION OF THE DRAWING FIGURES

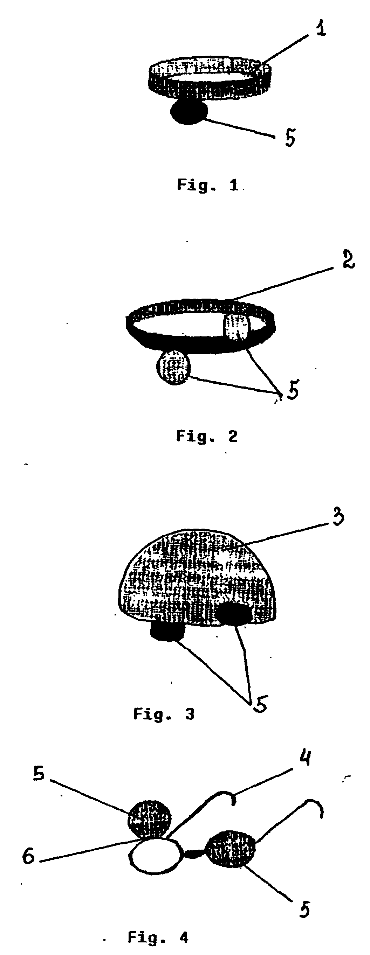

The accessory is explained by the drawings, in which:
Figure 1 is an embodiment of the system for fixing the accessory to the head of a user in the shape of a bandage;
Figure 2 ― the same in the shape of a hoop;
Figure 3 ― the same in the shape of a helmet;
Figure 4 ― the same in the shape of a spectacles rim;
Figure 5 is the accessory, fixed to the head of a user.
The examples shown on the drawings do not limit the variety of the embodiments of the accessory, but they are picked out as the most acceptable from the point of simplicity and low cost of manufacturing of this accessory. 'The accessory comprises the system for fixing it to the head of a user, made, for example, in the shape of an elastic bandage 1 (fig. 1), a hoop 2 (fig. 2), a helmet 3 (fig. 3) or a spectacles rim 4 (fig. 4). Several or at least one opaque flap or shield 5 is joint with the said system for fixing the accessory to the head of a user, said flap or shield 5 is made of light absorbing and/or scattering material and is joint, for example, with the spectacles rim 4 by means of a hinge.

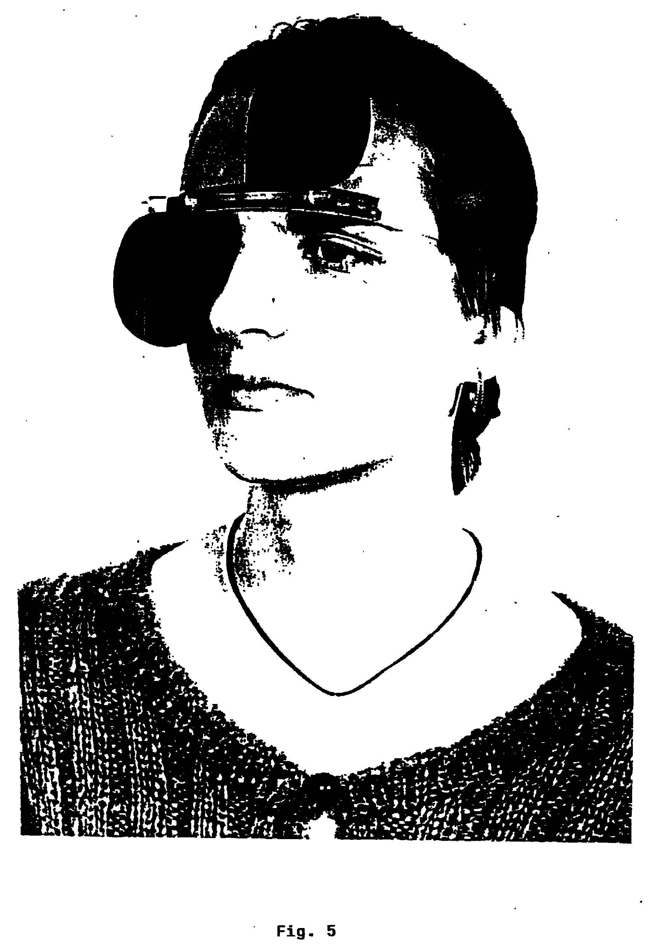

The system for fixing to the head of a user may be made of a fabric, resilient elastic band or rigid material in the embodiments of a hoop, a helmet or a spectacles rim. Such material may be plastic or light metal, for example, aluminum alloys. The shield or the flap may be also made of different materials, impeding the eye to see the image on the monitor screen. Such materials may be: plastic, glass, cartoon, fabric, wood, organic glass etc. Flap made of a soft material, for example, fabric is flexible, which is convenient in using. Also it is possible to combinative rigid and elastic materials for making the system for fixing the accessory to the head of a user, as it is shown on the fig. 5, where the front part of the said system is made of rigid transparent plastic, connected to an elastic resilient band, located at the back of the head and facilitating putting on and taking off of the accessory, and its fixing to the head of a user.

### DESCRIPTION OF PREFERRED EMBODIMENTS

The accessory is used in a following way: for example an elastic bandage 1 is fixed to the head of a user, a flap or a shield 5 for blocking one of the eyes is mounted opposite an eye and the user begins working with one of abovementioned devices, percepting an image with the other eye. On the expiry of a certain period (individual for each user) the elastic bandage 1 is replaced so that the field of vision of the other eye is blocked, and the first eye begins to percept the image, then the process repeats.

### INDUSTRIAL APPLICABILiTY

Worth mentioning that at present such electronic devices as computers, TV games and others, equipped with displays, have become widely spread both in business sphere and at home, including among children and teenagers, and because of this fact making it easy to work with such devices and increasing the efficiency of this work have become a vital necessity.

This accessory permits to increase in several times the duration of reading the text, percepting the image on a display, a screen of a monitor, TV-set and others electronic devices without eyes fatigue because it provides releasing the tension of the weakening mussels of both eyes, which is one of the main causes of the fatigue of users, working with computers and other devices.

## Claims

1. The personal accessory for working with the screen of an electronic device, comprising a system for fixing it to the head of a user, connected to a device for releasing the tension of the eyes, characterized with that the device for releasing the tension of the eyes is made in the shape of at least one member for blocking the field of vision of the eyes, which provides the possibility of blocking sequentially the user's left and right eyes.

2. The accessory according to the claim 1, characterized with that the member for blocking the field of vision of the eyes is made in the shape of a flap or a shield of light absorbing and/or scattering material.

3. The accessory according to the claim 2, characterized with that the shield has a hinge joint with the system for fixing the accessory to the head of a user.

4. The accessory according to any of the claims 1-3, characterized with that the system for fixing it to the head of a user is made in the shape of an elastic bandage.

5. The accessory according to any of the claims 1-3, characterized with that the system for fixing it to the head of a user is made in the shape of a hoop.

6. The accessory according to any of the claims 1-3, characterized with that the system for fixing it to the head of a user is made in the shape of a helmet.

7. The accessory according to any of the claims 1-3, characterized with that the system for fixing it to the head of a user is made in the shape of a spectacles rim.
